# FASCICULE DE BREVET EUROPEEN

(11) **EP 2 294 974 B1**
(45) Date de publication et mention de la délivrance du brevet: **07.12.2011**
(21) Numéro de dépôt: 10167580.9
(22) Date de dépôt: 28.06.2010
(51) Int. Cl.: A61B 5/00, A61F 5/01, A61F 13/08

(54) **Procédé de caractérisation d'une orthèse de contention veineuse élastique tricotée**
Verfahren zur Charakterisierung einer elastischen gestrickten Kompressionsorthese
Method for characterising an elastic knitted compressive orthosis

(30) Priorité: 01.09.2009 FR 0955958
(43) Date de publication de la demande: 16.03.2011
(73) Titulaire: LABORATOIRES INNOTHERA, 94110 Arcueil (FR)
(72) Inventeur: Bassez, Sophie, 91140, Villebon/Yvette (FR); Cros, François, 94200, Ivry/Seine (FR)
(74) Mandataire: Dupuis-Latour, Dominique

(56) Documents cités:
- EP-A1- 1 731 096
- WO-A1-98/58605
- WO-A1-2006/087442
- FR-A1- 2 797 393
- "PROGRAMME FINAL CAHIER DES RESUMES" CONGRES ANNUEL DE VASCULIS, XX, XX, 1 janvier 2004 (2004-01-01), pages 1-55, XP002342919

## Description

L'invention concerne les orthèses de compression veineuse élastique (CVE), qui sont indiquées dans les diverses manifestations cliniques d'insuffisance veineuse des membres inférieurs.

Ces orthèses, anciennement connues sous la dénomination de "bas de contention" ou "chaussettes de contention", sont des dispositifs médicaux textiles produisant un effet thérapeutique par compression/contention des membres inférieurs, par opposition aux "bas de maintien" (ou encore "bas de soutien" ou "bas anti-fatigue") et aux "bas mode" ou "chaussettes mode", qui ne sont pas des dispositifs médicaux à visée thérapeutique. Les orthèses de CVE sont conçues pour produire un effet thérapeutique par compression du membre inférieur sur une étendue plus ou moins grande, avec un profil dégressif vers le haut à partir de la cheville. Selon le type d'orthèse, la pression mesurée à la cheville peut varier de 10 à plus de 36 mmHg (soit 13 à 48 hPa, le mmHg étant toutefois d'usage courant comme unité de mesure de pression dans le domaine de la phlébologie et de la compression médicale). Les orthèses sont réparties selon le référentiel ASQUAL en quatre classes textiles, à savoir la classe I (13 à 20 hPa ≈ 10 à 15 mmHg à la cheville), la classe II (20 à 27 hPa ≈ 15 à 20 mmHg), la classe III (27 à 48 hPa ≈ 20 à 36 mmHg) et la classe IV (> 48 hPa ≈ > 36 mmHg).

La "pression" considérée ici, notamment pour la certification et la classification des orthèses de CVE auprès des autorités de santé nationales, a jusqu'à présent été la pression dite "pression textile" ou "pression de compression textile", telle que définie et calculée au sens de la norme française NF G30-120b, qui est le référentiel technique en vigueur en France pour ce type de produit (des normes semblables existant dans les autres pays européens, tel le référentiel allemand RAL-GZ387).

Cette technique de mesure de la pression consiste à enfiler l'orthèse sur une jambe-modèle de section circulaire normalisée formant gabarit métrologique, également connue sous la dénomination "modèle Hohenstein" et décrite à l'Annexe B de la norme NF G30-102b. Une fois l'orthèse enfilée sur la jambe-modèle, l'opérateur inscrit sur l'orthèse des repères à différentes altitudes de la jambe, par exemple à hauteur de la cheville, au départ du mollet, au niveau du mollet, au creux poplité, etc., jusqu'en haut de cuisse dans le cas d'un bas-cuisse ou d'un collant. L'orthèse est alors retirée de la jambe-modèle et soumise à un essai dynamométrique, en mettant sous tension l'orthèse entre deux mors dans la région préalablement repérée lors de l'enfilage sur la jambe-modèle.

La pression textile correspondante exercée par l'orthèse est évaluée à partir de la caractéristique tension/allongement obtenue par cet essai dynamométrique. On sait en effet que, pour une altitude donnée, la structure de la rangée de mailles de l'orthèse assure une répartition homogène des forces de rappel élastiques sur la circonférence de l'orthèse, c'est-à-dire le long d'un contour correspondant à une section horizontale du membre. L'application de ces forces de rappel élastiques sur le périmètre du contour engendre en un point donné, selon la loi de Laplace, une pression locale inversement proportionnelle au rayon de courbure du contour en ce point.

Le principe de calcul de la pression de contention repose donc sur l'application de la loi de Laplace, avec *P* = *T.c*, où *T* représente la tension linéique du textile dans le sens circonférentiel et c la courbure du contour où est appliquée la contention. Connaissant la tension *T* déterminée par la mesure dynamométrique, on peut ainsi en déduire la pression de textile de référence *P* exercée sur la section de jambe à l'altitude considérée. Cette méthode de laboratoire présente l'avantage d'être parfaitement rigoureuse et reproductible. Il s'agit d'une méthode de référence, sujette à très peu de biais dans la mesure où les paramètres métrologiques (jambe-modèle) et les conditions opératoires sont parfaitement définissables, et normalisés.

De ce fait, des mesures effectuées par des laboratoires différents ou par des opérateurs différents conduisent à des résultats semblables et reproductibles, utilisables notamment pour l'homologation des orthèses auprès des autorités sanitaires nationales.

Toutefois, il s'agit là de pressions théoriques, calculées à partir de mesures dynamométriques et dans l'hypothèse d'une jambe de section circulaire, qui ne reflètent pas nécessairement les pressions réellement exercées par les orthèses de CVE sur des jambes de patients réels, dont les sections de jambe sont loin d'être circulaires, et les morphologies très variées.

Pour une meilleure évaluation des orthèses de CVE et notamment de leur effet thérapeutique réel, on a tenté de mesurer la pression réellement exercée par l'orthèse en divers points du contour de la section de jambe d'un patient, à une altitude donnée.

Cette pression effective (qui n'est plus une pression moyenne mais une pression locale, exercée ponctuellement) est dénommée "pression d'interface", et correspond à la pression générée à l'interface de l'orthèse compressive avec la peau par la force de rappel des fils de trame élastiques constituant l'orthèse.

La détermination de la pression exercée localement sur un membre par une orthèse de CVE est un problème bien connu de l'état de la technique, comme cela ressort notamment des documents WO 2006/087442 A1 (Laboratoires Innothera), EP 1 731 096 A1 (Ganzoni Management AG) et WO 98/58605 A1 (Innothera Topic International), ou encore de la publication "Programme final - Cahier des résumés" du congrès annuel De vasculis 2004, Lyon 9-11 septembre 2004, pp. 1-55.

La pression d'interface dépend, toujours selon la loi de Laplace, de la tension du fil et de la courbure de la jambe. Mais en raison des différences de courbure, très variables selon la localisation anatomique, la pression d'interface exercée localement pourra varier dans de très grandes proportions.

En particulier, au niveau de la cheville, la section de jambe présente des variations importantes de courbure (au niveau du tendon d'Achille, des malléoles, des creux rétro-malléolaires, etc.), et des études ont montré que la pression d'interface peut varier dans de très grandes proportions d'un point à l'autre de ce contour, par exemple de 20 mm Hg au niveau des creux rétro-malléolaires jusqu'à près de 90 mm Hg au niveau de la région du tendon d'Achille, soit un facteur de variation de près de x4,5. Ces variations résultent de la loi de Laplace : à une structure anatomique de petit rayon (forte courbure) correspond une pression plus élevée que pour une structure anatomique de plus grand rayon (faible courbure).

De plus, au cours du temps la forme de la jambe varie sous l'influence de la position du sujet et de l'activité musculaire : lors d'une contraction musculaire, il y a réorganisation de la masse musculaire avec localement modification de la forme de la jambe à une altitude donnée, et donc de la courbure du contour et de sa circonférence. La tension exercée par l'orthèse de CVE va s'en trouver modifiée, et par voie de conséquence la pression d'interface, à la fois du fait de la variation de tension de l'orthèse (conséquence de la variation du périmètre) et du fait de la modification de la courbure de la jambe au niveau du site de mesure (conséquence de la réorganisation de la masse musculaire).

La pression d'interface est donc notablement influencée par de multiples facteurs liés à la morphologie des sujets :
- variabilité selon le site de mesure sur la jambe, chez un même sujet ;
- dépendance avec la posture et l'activité du sujet ;
- variabilité des morphologies, et donc des courbures et des mensurations entre individus, pour un même site ou un même niveau de mesure.

La pression d'interface étant très sensible à ces paramètres, si l'on souhaite utiliser cette grandeur comme critère d'évaluation des orthèses, une très grande rigueur sera nécessaire dans le protocole de mesure et dans le choix du groupe-témoin (panel) de patients, afin de garantir une cohérence des résultats en termes de reproductibilité et comparabilité.

Le choix des sites de mesure, leur localisation ainsi que la posture du sujet devront être précis, sans ambiguïté et consensuels. Par ailleurs, étant donné la variabilité inter-individuelle, le nombre de sujets constituant le groupe-témoin devra être grand, et ces derniers devront être choisis en fonction des différentes tailles d'orthèses disponibles, de manière à ce que les résultats soient représentatifs en termes de mensurations pour tout intervalle de taille d'orthèse.

En tout état de cause, il n'existe pour le moment aucun consensus sur le choix des sites de mesure sur la section de jambe ni sur les conditions de mesure (position du sujet, situation statique ou dynamique, etc.).

De plus, outre la difficulté de réunir les conditions ci-dessus, l'évaluation de la pression d'interface se heurte à un problème supplémentaire, qui est sa très grande dépendance au choix du capteur et au protocole de mesure.

On connaît divers types de capteurs permettant de mesurer la force perpendiculaire ou la pression exercée sur le capteur à l'interface entre la peau et l'orthèse compressive. Les principales technologies employées sont : capteurs pneumatiques et électropneumatiques, capteurs à déplacement de fluide, capteurs résistifs et capteurs capacitifs. Et dans ces diverses catégories, plusieurs modèles sont disponibles, chacun avec ses particularités de mise en oeuvre.

Le choix d'un capteur pour mesurer la pression d'interface doit répondre à de multiples considérations, non seulement métrologiques mais également pratiques (simplicité de mise en oeuvre, facilité d'utilisation et possibilité d'enregistrer les mesures, etc.).

Les principales recommandations sont les suivantes :
- finesse de la sonde, afin de répondre à un critère épaisseur/diamètre inférieur à 1/10 : en effet, la pression à mesurer ne doit pas être perturbée par la présence du capteur, ce qui impose de rechercher un compromis entre la résolution désirée et le gradient de pression à évaluer ; de plus l'épaisseur de la sonde doit être suffisamment faible pour ne pas perturber la mesure de pression lorsqu'elle est introduite entre l'orthèse et la surface de la jambe ;
- possibilité pour la sonde de se conformer à la morphologie de la jambe sans être sensible à la courbure : si le capteur n'est pas suffisamment flexible, il se conformera mal au galbe de la jambe, et la courbure de la partie sensible du capteur risque de modifier la réponse, notamment sur des zones de forte courbure de la jambe, sujette à des erreurs d'évaluation notables ;
- étendue de mesure et précision compatible avec les ordres de grandeur des pressions à mesurer ;
- possibilité de faire des mesures en continu à une fréquence d'échantillonnage adéquate.

Concrètement, il apparaît qu'aucun capteur actuellement disponible ne remplit toutes les conditions requises de façon satisfaisante.

Par voie de conséquence, la mesure *in vivo* de la pression d'interface apparaît aujourd'hui trop sensible à de nombreux facteurs et biais divers pour pouvoir constituer une méthode de référence acceptable et représentative, susceptible d'un consensus large permettant d'aboutir à un protocole standardisé.

Le but de la présente invention est de proposer une alternative aux techniques de mesure *in vivo* de la pression d'interface dont on vient d'exposer les limites et les difficultés de mise en oeuvre.

L'alternative proposée par l'invention présente, comme on le verra, un caractère parfaitement reproductible, tout en tenant compte de la variabilité morphologique des jambes de patient - à la différence des techniques de mesure de la pression textile, qui reposent sur l'hypothèse théorique d'une jambe de section circulaire, très éloignée de la réalité.

En d'autres termes, le but de l'invention est de proposer une méthode alternative combinant les avantages des deux méthodes connues, à savoir essentiellement la représentativité de la morphologie (dans le cas de la mesure *in vivo* de la pression d'interface) et la standardisation et la répétibilité du protocole de mesure (dans le cas des mesures de laboratoire de la pression textile).

Le point de départ de l'invention est l'étude du point de savoir s'il est possible de trouver une relation entre la pression textile (mesures normalisées en laboratoire) et la pression d'interface (mesures *in vivo*).

En effet, pour s'affranchir des problèmes de mesure liés à la détermination du groupe-témoin de sujets et à la variabilité morphologique individuelle, une solution serait d'établir une relation entre les pressions *in vivo* appliquées sur une section de jambe à une altitude donnée, et la pression textile théorique correspondant à cette même section de jambe.

On ne sait pas a *priori* relier théoriquement la pression théorique textile aux pressions mesurées *in vivo,* du fait de la très grande variabilité interindividus. Toutefois, des études statistiques ont permis de constater que la moyenne des pressions d'interfaces, mesurées *in vivo,* exercées par une orthèse CVE sur une section de jambe à une altitude donnée est corrélée à la pression textile exercée sur cette même section de jambe. L'invention propose, partant de cette constatation, d'utiliser la pression textile - que l'on sait évaluer de façon standardisée - pour extrapoler une valeur de pression d'interface moyenne exercée sur une section de jambe, et d'utiliser cette pression d'interface moyenne (dont on a montré qu'elle est corrélée à la pression textile) pour caractériser l'orthèse de CVE, notamment à des fins de définition des classes de contention et de certification auprès des autorités officielles.

Des études statistiques montrent que l'extrapolation peut se faire simplement, par application à la valeur de pression textile d'un coefficient multiplicateur prédéterminé, supérieur à l'unité, qui peut être soit un coefficient global pour la jambe, soit un coefficient particulier, correspondant à une altitude donnée d'une section de jambe - typiquement, la section considérée au niveau de la cheville.

Une telle manière de procéder permet de disposer d'une méthode d'évaluation de la pression qui soit répétable, standardisée, et qui prenne en compte la variabilité morphologique, sans les inconvénients des mesures *in vivo.* Par un choix approprié du coefficient, une correction standardisée est possible, pour chaque section de jambe, pour passer de la pression textile à la pression d'interface moyenne.

Plus précisément, la présente invention propose un procédé de caractérisation d'une orthèse de contention veineuse élastique tricotée apte à exercer en direction circonférentielle une force de rappel élastique propre à produire une compression de la jambe à un niveau de pression thérapeutique. Cette pression est exercée localement par l'orthèse en une pluralité correspondante de points répartis le long d'un contour d'une section de la jambe du patient, à une altitude donnée de cette jambe.

De façon caractéristique, le procédé comprend les étapes suivantes :
a) détermination du périmètre dudit contour de la section de jambe de patient à ladite altitude donnée ;
b) mesure dynamométrique de la pression textile susceptible d'être exercée par l'orthèse sur une section circulaire de périmètre égal à la valeur calculée à l'étape a) ;
c) application à la valeur de pression textile mesurée à l'étape b) d'un coefficient multiplicateur prédéterminé, supérieur à l'unité ; et
d) utilisation de la valeur de pression déterminée à l'étape c) comme valeur estimée d'une pression d'interface moyenne exercée sur la section de jambe de patient à ladite altitude donnée.

La valeur du coefficient prédéterminé peut être comprise dans l'intervalle [1,138 - 1,145] s'étendant autour de la valeur 1,142, indépendamment de l'altitude du contour de la section de jambe.

La valeur du coefficient prédéterminé peut également être fonction de l'altitude du contour de la section de jambe.

La mesure dynamométrique de la pression textile de l'étape b) peut en particulier être réalisée selon la norme française NF G 30-102b.

Selon un autre aspect du procédé, celui-ci comprend une étape préalable de détermination dudit coefficient multiplicateur, comprenant les sous-étapes suivantes :
- constitution d'un échantillon représentatif de patients ;
- scannage des jambes des patients de cet échantillon, de manière à déterminer, pour chaque jambe, la forme du contour d'au moins une section définie à une altitude donnée de cette jambe ;
- détermination des caractéristiques dimensionnelles et rhéologiques de l'orthèse à ladite altitude donnée ;
- calcul des courbures du contour de jambe en une pluralité correspondante de points répartis le long de ce contour de jambe ;
- évaluation, par un calcul à partir des valeurs des courbures du contour de jambe et des caractéristiques dimensionnelles et rhéologiques de l'orthèse, des pressions exercées localement par l'orthèse sur ladite section en chacun desdits points du contour de jambe ;
- calcul, à partir des valeurs desdites pressions exercées localement, d'une valeur moyenne de pression d'interface exercée sur l'ensemble des points du contour de jambe ;
- calcul d'une courbure de référence, ladite courbure de référence étant celle d'un cercle de périmètre égal au périmètre dudit contour de jambe ;
- évaluation, par un calcul à partir de la valeur de la courbure de référence et des caractéristiques dimensionnelles et rhéologiques de l'orthèse, de la pression textile susceptible d'être exercée par l'orthèse sur une section circulaire correspondant à ladite courbure de référence ; et
- détermination d'un ratio entre la valeur moyenne de la pression d'interface et la pression textile ainsi calculée, pour donner ledit coefficient multiplicateur prédéterminé.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue en élévation d'une jambe, montrant les différents niveaux normalisés auxquels sont mesurées les pressions de compression susceptibles d'être appliquées par une orthèse de CVE.
La Figure 2 est une coupe prise au niveau de la cheville, illustrant comment varie le long du contour de la section de jambe la pression d'interface localement appliquée par l'orthèse.
La Figure 3 est un schéma illustrant les différentes étapes du procédé de l'invention.

Lorsqu'une orthèse de CVE est enfilée sur un membre, le textile tendu exerce une compression résultant de la force de rappel des fibres élastiques qui composent le matériau de l'orthèse. Pour une même dimension à l'état non tendu, c'est-à-dire une même section circulaire, avec un matériau qui présente un coefficient d'élasticité supérieure (ou "renforcée"), l'enfilage sur le membre produit une force de rappel plus élevée, donc une compression plus importante se manifestant localement par une composante normale de pression plus élevée exercée à la surface du membre. Le choix de la maille et des fils, ainsi que le dimensionnement des différentes rangées de maille, sont définis de manière à appliquer à différentes altitudes de la jambe des pressions prédéterminées, définies pour chaque classe de contention/compression en référence à des gabarits métrologiques tels que le modèle Hohenstein.

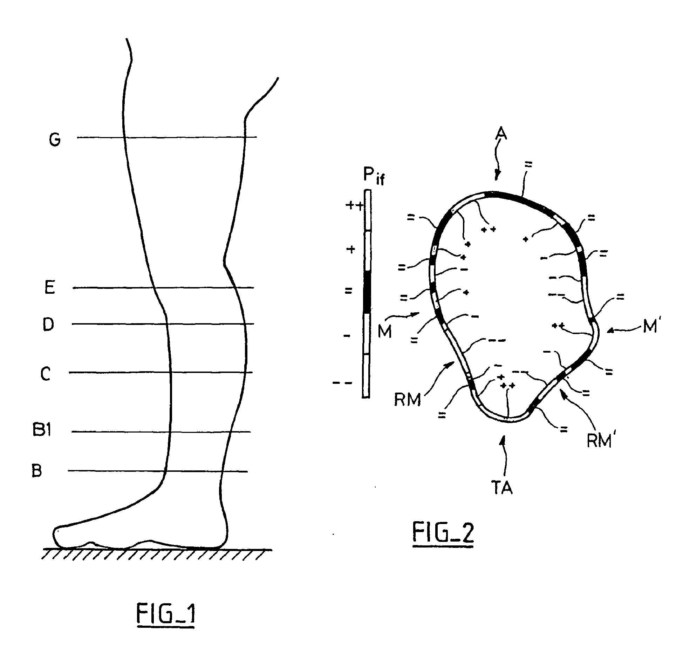

Sur la figure 1, on a représenté les diverses altitudes normalisées de la jambe telles que définies par ce modèle Hohenstein, à savoir :
- *B* :: au périmètre minimal de la cheville, soit à une altitude *z* = 12 cm par rapport au sol,
- *B1* :: à la naissance du tendon d'Achille, à *z* = 20 cm,
- *C* :: au périmètre maximal du mollet, à *z* = 31 cm,
- *D* :: au-dessous du genou, à *z* = 39 cm,
- *E* :: au creux poplité, à *z* = 45 cm,
- *F* :: en milieu de cuisse, à *z* = 60 cm,
- *G* :: en haut de cuisse, à *z* = 72 cm.

La pression exercée à l'altitude du point *B* du modèle Hohenstein, c'est-à-dire au niveau de la cheville, est la pression prescrite pour la classe de contention/compression choisie (I, II, III ou IV) cette pression correspondant par ailleurs à la valeur de pression (théoriquement) la plus élevée exercée sur le membre.

Pour une altitude donnée, la structure de la rangée de mailles assure une répartition homogène des forces de rappel élastique sur la circonférence du bas, c'est-à-dire le long d'un contour correspondant à une section horizontale du membre. L'application de ces forces de rappel élastique sur le périmètre du contour engendre en un point donné, selon la loi de Laplace, une pression locale inversement proportionnelle au rayon de courbure du contour en ce point.

Sur la Figure 2, on a représenté la pression d'interface localement appliquée par l'orthèse le long du contour d'une section de jambe prise au niveau de la cheville. On reconnaît sur ce contour les régions malléolaires M, M', du tendon d'Achille TA, des creux rétro-malléolaires RM, RM' et la région de la crête antérieure A. On peut observer la très grande variabilité des courbures le long de ce contour, qui entraîne une variabilité correspondante (du fait de la loi de Laplace) des pressions d'interface localement exercées en chaque point de ce contour. Par rapport à la valeur moyenne de la pression d'interface, on a indiqué par "+" et "++" les régions où la pression est supérieure à cette moyenne, et par "-" et "--" celles où elle est inférieure, les régions repérées "=" correspondant à des valeurs de pression d'interface situées autour de la valeur moyenne. On constate ainsi des variations très importantes, et rapides, de la pression d'interface le long du contour, par exemple entre la région du tendon d'Achille TA et celle des creux rétro-malléolaires RM et RM'. Comme on l'a indiqué plus haut, entre les valeurs extrêmes le facteur de variation peut atteindre x4,5.

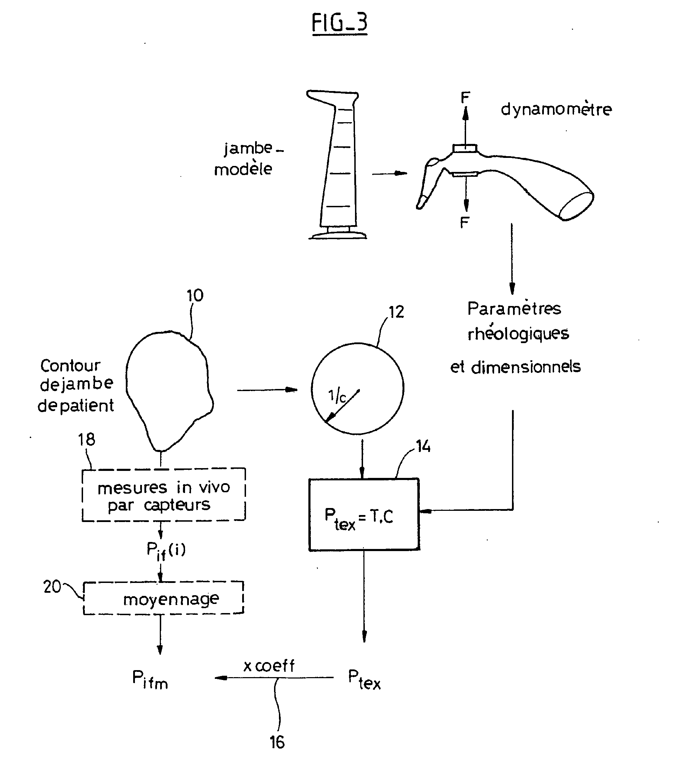

Le principe de l'invention, illustré Figure 3, consiste à remplacer la mesure directe (*in vivo*) des pressions d'interface - mesure très délicate compte tenu de la très grande variabilité morphologique et de la difficulté de trouver des capteurs procurant des résultats significatifs et reproductibles - par une évaluation indirecte, selon une technique reproductible et simple à mettre en oeuvre permettant d'estimer, sans la mesurer, la pression d'interface susceptible d'être appliquée par l'orthèse sur une section de jambe à une altitude donnée.

Cette méthode indirecte consiste, partant d'un contour réel de jambe de patient 10, à mesurer le périmètre de ce contour (ce qui peut être fait de façon très précise, à partir d'une représentation numérique de la jambe obtenue par scannage) et à définir un cercle 12 de même périmètre que celui du contour de jambe 10.

L'étape suivante consiste à déterminer la pression textile *Pₜₑₓ* qui serait appliquée par l'orthèse sur une section de jambe circulaire correspondant à ce cercle 12. Cette détermination peut être faite simplement (bloc 14) par application de la loi de Laplace, connaissant d'une part la courbure c du cercle 12 (*c* = *1*/*R*) et d'autre part la tension linéique *T* qu'exercerait l'orthèse dans cette configuration. Cette tension linéique *T* est déterminée à partir de résultats de mesures dynamométriques en laboratoire, sur la base d'un gabarit métrologique de jambe-modèle et d'un protocole tel que celui décrit dans la norme française NF G30-102b, le tout permettant d'obtenir de façon extrêmement précise et reproductible les paramètres rhéologiques et dimensionnels d'une orthèse donnée.

Une fois la pression textile *Pₜₑₓ* ainsi déterminée, l'étape suivante (étape 16) consiste à appliquer à cette valeur *Pₜₑₓ* un coefficient prédéterminé *coeff*, pour donner une valeur estimée *P_{ifm}* de pression d'interface moyenne, qui pourra être utilisée pour caractériser l'orthèse.

Cette évaluation indirecte de la pression d'interface moyenne *P_{ifm}* permet d'éviter le recours à des mesures *in vivo* (bloc 18) au moyen de capteurs donnant des valeurs de pression d'interface locales *P_{if}*(*i*) qu'il conviendrait ensuite de moyenner (bloc 20) pour obtenir la valeur de pression d'interface moyenne *P_{ifm}* recherchée.

La méthode selon l'invention (étapes 10, 12, 14, 16) est simple, reproductible et aisément normalisable, à la différence de la méthode directe (étapes 10, 18, 20) sujette, comme on l'a expliqué plus haut, à de nombreux biais et difficultés de mise en oeuvre.

La détermination du coefficient *coeff* à utiliser résulte d'une étude statistique, réalisée une fois pour toutes.

À cet effet, on a utilisé les résultats de 100 jambes scannées correspondant à un groupe-témoin représentatif en termes de sexe, âge et indice de masse corporelle (IMC). Ces jambes peuvent être scannées notamment par une installation de pléthysmographie laser telle que celle décrite dans les FR 2 774 276 A1 et FR 2 804 595 A1 (Innothéra Topic International), technique qui permet d'établir une cartographie très précise du membre d'un sujet le long de sections successives de ce membre.

Pour chaque section considérée du membre scanné, tout particulièrement pour les sections normalisées B, B1, C, D, E et G, on a calculé la courbure en 72 points de chaque contour. On a également déterminé la courbure de référence c correspondant à ce contour, c'est-à-dire la courbure d'un cercle qui aurait même périmètre que la section de jambe à l'altitude considérée.

On a ensuite calculé, pour chaque section, la pression d'interface exercée en chacun des points considérés. Ce calcul peut notamment être effectué par une technique telle que celle décrite par le WO 2004/095342 A2 (Laboratoires Innothéra), qui explique la manière dont il est possible de combiner les données morphologiques des jambes (déterminées par scannage) aux données rhéologiques propres à l'orthèse (déterminées par mesures dynamométriques), pour produire une cartographie complète des pressions appliquées sur le membre, et notamment une cartographie des pressions d'interface *P_{if}*(*i*), avec *i* = 1 ... 72, exercées en chacun des points du contour, cartographie correspondant par exemple à celle illustrée sur la Figure 2.

À partir de ces valeurs *P_{if}*(*i*) de pression d'interface locale, on a déterminé ensuite la pression moyenne correspondante *P_{ifm}.*

On a déterminé par ailleurs, par application de la loi de Laplace *Pₜₑₓ* = *T*.*c*, la pression textile *Pₜₑₓ* qu'exercerait l'orthèse sur une section circulaire dont le rayon correspond à la courbure de référence c.

Si l'on détermine le ratio *P_{ifm}*/*Pₜₑₓ* globalement pour toutes les sections de jambe et pour tous les sujets considérés, on obtient un résultat *P_{ifm}*/*Pₜₑₓ* = 1,142, valeur centrée sur un intervalle [1,138-1,145] correspondant à un intervalle de confiance à 95 %. Ceci permet de conclure à l'équivalence, sur l'ensemble de la population et avec une marge de 14,2 %, entre la pression d'interface moyenne réellement exercée par l'orthèse et la pression textile mesurée en laboratoire.

Il est possible de calculer le même ratio *P_{ifm}*/*Pₜₑₓ* spécifiquement pour chacune des sections normalisées B, B1, C, D, E et G, ce qui donne des valeurs correspondantes de coefficients et d'intervalles de confiance.

En variante, il est possible de prévoir des coefficients différents, toujours déterminés de la même manière, en fonction des sous-populations de patients, selon le sexe (coefficients différents pour un homme et une femme), selon la classe d'âge, selon l'indice de masse corporelle, etc. de manière à obtenir, par estimation indirecte, une valeur de pression d'interface Pif la plus proche possible de la pression réelle qui pourrait être mesurée *in vivo.*

## Revendications

1. Un procédé de caractérisation d'une orthèse de contention veineuse élastique tricotée apte à exercer en direction circonférentielle une force de rappel élastique propre à produire une compression de la jambe à un niveau de pression thérapeutique,
ladite pression étant exercée localement par l'orthèse en une pluralité correspondante de points répartis le long d'un contour d'une section de la jambe du patient, à une altitude donnée de cette jambe,
procédé **caractérisé par** les étapes suivantes :
a) détermination du périmètre dudit contour de la section de jambe de patient (10) à ladite altitude donnée ;
b) mesure dynamométrique de la pression textile (*Pₜₑₓ*) susceptible d'être exercée par l'orthèse sur une section circulaire (12) de périmètre égal à la valeur calculée à l'étape a) ;
c) application (16) à la valeur de pression textile mesurée à l'étape b) d'un coefficient multiplicateur prédéterminé (*coeff*), supérieur à l'unité ; et
d) utilisation de la valeur de pression déterminée à l'étape c) comme valeur estimée d'une pression d'interface moyenne (*P_{ifm}*) exercée sur la section de jambe de patient à ladite altitude donnée.

2. Le procédé de la revendication 1, dans lequel la valeur dudit coefficient prédéterminé est une valeur comprise dans l'intervalle [1,138 - 1,145] s'étendant autour de la valeur 1,142, indépendamment de l'altitude du contour de la section de jambe.

3. Le procédé de la revendication 1, dans lequel la valeur dudit coefficient prédéterminé est une valeur fonction de ladite altitude du contour de la section de jambe.

4. Le procédé de la revendication 1, dans lequel la mesure dynamométrique de la pression textile de l'étape b) est réalisée conformément à la norme française NF G 30-102b.

5. Le procédé de la revendication 1, comprenant en outre une étape préalable de détermination dudit coefficient multiplicateur, comprenant les sous-étapes suivantes :
- constitution d'un échantillon représentatif de patients ;
- scannage des jambes des patients de cet échantillon, de manière à déterminer, pour chaque jambe, la forme du contour d'au moins une section définie à une altitude donnée de cette jambe ;
- détermination des caractéristiques dimensionnelles et rhéologiques de l'orthèse à ladite altitude donnée ;
- calcul des courbures du contour de jambe en une pluralité correspondante de points répartis le long de ce contour de jambe ;
- évaluation, par un calcul à partir des valeurs des courbures du contour de jambe et des caractéristiques dimensionnelles et rhéologiques de l'orthèse, des pressions exercées localement par l'orthèse sur ladite section en chacun desdits points du contour de jambe ;
- calcul, à partir des valeurs desdites pressions exercées localement, d'une valeur moyenne de pression d'interface exercée sur l'ensemble des points du contour de jambe ;
- calcul d'une courbure de référence, ladite courbure de référence étant celle d'un cercle de périmètre égal au périmètre dudit contour de jambe ;
- évaluation, par un calcul à partir de la valeur de la courbure de référence et des caractéristiques dimensionnelles et rhéologiques de l'orthèse, de la pression textile susceptible d'être exercée par l'orthèse sur une section circulaire correspondant à ladite courbure de référence ; et
- détermination d'un ratio entre la valeur moyenne de la pression d'interface et la pression textile ainsi calculée, pour donner ledit coefficient multiplicateur prédéterminé.

## Claims

1. A method for characterizing a knitted elastic venous restraint orthosis able to exert in a circumferential direction an elastic restoring force suitable for producing a compression of the leg with a therapeutic pressure level,
the said pressure being exerted locally by the orthosis at a corresponding plurality of points distributed along a contour of a cross-section of the leg of the patient, at a given altitude of this leg,
method **characterized by** the following steps:
a) determination of the perimeter of the said contour of the patient leg cross-section (10) at the said given altitude;
b) dynamometric measurement of the textile pressure (*Pₜₑₓ*) liable to be exerted by the orthosis on a circular cross-section (12) of perimeter equal to the value calculated in step a);
c) application (16) to the textile pressure value measured in step b) of a predetermined multiplier coefficient (coeff), greater than unity; and
d) use of the pressure value determined in step c) as estimated value of a mean interface pressure (*P_{ifm}*) exerted on the patient leg cross-section at the said given altitude.

2. The method of Claim 1, in which the value of the said predetermined coefficient is a value included in the interval [1.138 - 1.145] extending around the value 1.142, independently of the altitude of the contour of the leg cross-section.

3. The method of Claim 1, in which the value of the said predetermined coefficient is a value dependent on the said altitude of the contour of the leg cross-section.

4. The method of Claim 1, in which the dynamometric measurement of the textile pressure of step b) is carried out in accordance with French standard NF G 30-102b.

5. The method of Claim 1, furthermore comprising a prior step of determining the said multiplier coefficient, comprising the following sub-steps:
- construction of a representative sample of patients;
- scanning of the legs of the patients of this sample, so as to determine, for each leg, the shape of the contour of at least one defined cross-section at a given altitude of this leg;
- determination of the dimensional and rheological characteristics of the orthosis at the said given altitude;
- calculation of the curvatures of the leg contour at a corresponding plurality of points distributed along this leg contour;
- evaluation, by a calculation on the basis of the values of the curvatures of the leg contour and of the dimensional and rheological characteristics of the orthosis, of the pressures exerted locally by the orthosis on the said cross-section at each of the said points of the leg contour;
- calculation, on the basis of the values of the said locally exerted pressures, of a mean value of interface pressure exerted on the set of points of the leg contour;
- calculation of a reference curvature, the said reference curvature being that of a circle of perimeter equal to the perimeter of the said leg contour;
- evaluation, by a calculation on the basis of the value of the reference curvature and of the dimensional and rheological characteristics of the orthosis, of the textile pressure liable to be exerted by the orthosis on a circular cross-section corresponding to the said reference curvature; and
- determination of a ratio of the mean value of the interface pressure to the textile pressure thus calculated, so as to yield the said predetermined multiplier coefficient.

## Patentansprüche

1. Verfahren zur Charakterisierung einer gewirkten elastischen Venenfixierungsprothese, die in Umfangsrichtung eine elastische Rückstellkraft ausüben kann, die eine Kompression des Beins mit einem therapeutischen Druckniveau erzeugen kann,
wobei der Druck durch die Prothese lokal an mehreren entsprechenden Punkten, die längs eines Umrisses eines Abschnitts des Beins des Patienten verteilt sind, auf einer gegebenen Höhe dieses Beins ausgeübt wird,
wobei das Verfahren **gekennzeichnet ist durch** die folgenden Schritte:
a) Bestimmen des Umfangs des Umrisses des Abschnitts des Beins des Patienten (10) auf der gegebenen Höhe;
b) dynamometrisches Messen des Textildrucks (Pₜₑₓ), der **durch** die Prothese auf einen kreisförmigen Abschnitt (12) mit einem Umfang, der gleich dem im Schritt a) berechneten Wert ist, ausgeübt werden kann;
c) Anwenden (16) eines vorgegebenen Multiplikationskoeffizienten (coeff), der größer als eins ist, auf den im Schritt b) gemessenen Wert des Textildrucks; und
d) Verwenden des im Schritt c) bestimmten Druckwerts als geschätzten Wert eines mittleren Grenzflächendrucks (P_{ifm}), der auf den Abschnitt des Beins des Patienten auf der gegebenen Höhe ausgeübt wird.

2. Verfahren nach Anspruch 1, wobei der Wert des vorgegebenen Koeffizienten ein Wert ist, der unabhängig von der Höhe des Umrisses des Abschnitts des Beins in dem Intervall [1,138-1,145] liegt, das um den Wert 1,142 angeordnet ist.

3. Verfahren nach Anspruch 1, wobei der Wert des vorgegebenen Koeffizienten ein Wert ist, der von der Höhe des Umrisses des Abschnitts des Beins abhängt.

4. Verfahren nach Anspruch 1, wobei die dynamometrische Messung des Textildrucks im Schritt b) gemäß der französischen Norm NF G 30-102b verwirklicht ist.

5. Verfahren nach Anspruch 1, das außerdem einen vorherigen Schritt des Bestimmens des Multiplikationskoeffizienten umfasst, der die folgenden Unterschritte umfasst:
- Bilden einer repräsentativen Stichprobe von Patienten;
- Abtasten der Beine der Patienten dieser Stichprobe in der Weise, dass für jedes Bein die Form des Umrisses wenigstens eines Abschnitts, der auf einer gegebenen Höhe dieses Beins definiert ist, bestimmt wird;
- Bestimmen der dimensionalen und der rheologischen Charakteristiken der Prothese auf der gegebenen Höhe;
- Berechnen von Krümmungen des Umrisses des Beins an mehreren entsprechenden Punkten, die längs dieses Umrisses des Beins verteilt sind;
- Ermitteln der Drücke, die durch die Prothese auf den Abschnitt an jedem dieser Punkte des Umrisses des Beins lokal ausgeübt werden, durch eine Berechnung anhand dieser Krümmungswerte des Umrisses des Beins und der dimensionalen und rheologischen Charakteristiken der Prothese;
- Berechnen eines mittleren Werts des Grenzflächendrucks, der auf die Gesamtheit der Punkte der Umrisse des Beins ausgeübt wird, ausgehend von den Werten dieser lokal ausgeübten Drücke;
- Berechnen einer Referenzkrümmung, wobei die Referenzkrümmung jene eines Kreises mit einem Umfang ist, der gleich dem Umfang des Umrisses des Beins ist;
- Ermitteln des Textildrucks, der von der Prothese auf einen kreisförmigen Abschnitt ausgeübt werden kann, der der Referenzkrümmung entspricht, durch eine Berechnung ausgehend von dem Wert der Referenzkrümmung und den dimensionalen und rheologischen Charakteristiken der Prothese; und
- Bestimmen eines Verhältnisses zwischen dem Mittelwert des Grenzflächendrucks und dem somit berechneten Textildruck, um den vorgegebenen Multiplikationskoeffizienten zu erhalten.
